# FASCICULE DE BREVET EUROPEEN

(11) **EP 2 288 731 B1**
(45) Date de publication et mention de la délivrance du brevet: **20.08.2014**
(21) Numéro de dépôt: 09757698.7
(22) Date de dépôt: 04.06.2009
(51) Int. Cl.: C12Q 1/68

(54) **MÉTHODE D'IDENTIFICATION ÉLECTROCHIMIQUE DE SÉQUENCES CIBLES DE NUCLÉOTIDES**
VERFAHREN ZUR ELEKTROCHEMISCHEN IDENTIFIZIERUNG VON ZIELNUKLEOTIDSEQUENZEN
METHOD FOR ELECTROCHEMICALLY IDENTIFYING TARGET NUCLEOTIDE SEQUENCES

(30) Priorité: 05.06.2008 FR 0803143
(43) Date de publication de la demande: 02.03.2011
(73) Titulaire: Université Paris Diderot - Paris 7, 75205 Paris Cedex 13 (FR); Centre National de la Recherche Scientifique (CNRS), 75016 Paris (FR)
(72) Inventeur: LIMOGES, Benoît, F-91220 Brétigny-sur-Orge (FR); DEFEVER, Thibaut, F-59310 Orchies (FR); MARCHAL, Damien, F-75009 Paris (FR)
(74) Mandataire: Takeuchi, Maya
(86) Numéro de dépôt international: PCT/FR2009/000653
(87) Numéro de publication internationale: WO 2009/147322

(56) Documents cités:
- WO-A-2007/099236
- US-B1- 6 833 267
- BOON E M ET AL: "Mutation detection by electrocatalysis at DNA-modified electrodes" NATURE BIOTECHNOLOGY, NATURE PUBLISHING GROUP, NEW YORK, NY, US, vol. 18, no. 10, 1 octobre 2000 (2000-10-01), pages 1096-1100, XP002400609 ISSN: 1087-0156
- MILLAN K M ET AL: "SEQUENCE-SELECTIVE BIOSENSOR FOR DNA BASED ON ELECTROACTIVE HYBRIDIZATION INDICATORS" ANALYTICAL CHEMISTRY, AMERICAN CHEMICAL SOCIETY. COLUMBUS, US, vol. 65, 1 janvier 1993 (1993-01-01), pages 2317-2323, XP000396986 ISSN: 0003-2700
- MARUYAMA K ET AL: "Electrochemical and DNA-binding properties of dipyridophenazine complexes of osmium(II)", JOURNAL OF THE ELECTROANALYTICAL CHEMISTRY, LAUSANNE, CH, vol. 510, no. 1-2, 1 January 2001 (2001-01-01), pages 96-102, XP002407812, ISSN: 0368-1874, DOI: 10.1016/S0022-0728(01)00549-6
- YOON-JUNG JANG ET AL: "Comparison of the Binding Modes of [Ru(2,2'-bipyridine) 3 ]2+", BULLETIN OF THE KOREAN CHEMICAL SOCIETY, vol. 31, no. 5, 20 May 2010 (2010-05-20), pages 1314-1318, XP055100843, ISSN: 0253-2964, DOI: 10.5012/bkcs.2010.31.5.1314
- YU H J ET AL: "Synthesis, DNA-binding and photocleavage studies of ruthenium complexes [Ru(bpy)2(mitatp)]<2+> and [Ru(bpy)2(nitatp)]<2+>", JOURNAL OF INORGANIC BIOCHEMISTRY, ELSEVIER INC, US, vol. 103, no. 6, 1 June 2009 (2009-06-01), pages 881-890, XP026127263, ISSN: 0162-0134, DOI: 10.1016/J.JINORGBIO.2009.03.005 [retrieved on 2009-03-25]
- LEE ET AL: "DNA biosensor based on the electrochemiluminescence of Ru(bpy)3<2+> with DNA-binding intercalators", BIOELECTROCHEMISTRY, ELESEVIER, AMSTERDAM, NL, vol. 70, no. 2, 3 April 2007 (2007-04-03), pages 228-234, XP022015476, ISSN: 1567-5394, DOI: 10.1016/J.BIOELECHEM.2006.09.003

## Description

La présente invention se rapporte à une méthode et à un ensemble d'identification électrochimique de séquences cibles de nucléotides.

Un domaine d'application envisagé, est notamment celui de l'analyse rapide d'échantillons biologiques susceptibles de contenir des bactéries ou des virus, et plus généralement tout acide nucléique.

Des méthodes de détection électrochimique connues permettent déjà de mettre en évidence des séquences cibles d'acides nucléiques. Certaines font appel à la fois à des procédés d'amplification des acides nucléiques, par exemple de type «PCR » pour *Polymerase chain reaction* en langue anglaise et à des procédés de voltampérométrie cyclique. Selon l'une de ces méthodes, on fournit un échantillon biologique renfermant un acide nucléique, lequel présente une séquence cible de nucléotides déterminée, et on ajoute à l'échantillon biologique un agent oxydant apte à oxyder au moins l'une des bases nucléotidiques de la séquence cible. Les moyens d'amplification comprennent des nucléotides d'un type incluant la base nucléotidique oxydable et les nucléotides sont bien évidemment destinés à être consommés durant la mise en oeuvre du procédé d'amplification pour produire des séquences d'acides nucléiques répliqués. Simultanément, ou après chaque étape d'amplification, on applique un champ électrique à l'échantillon pour provoquer la réaction de l'agent oxydant avec la base nucléotidique oxydable et on mesure le courant électrique qui traverse en conséquence l'échantillon. Selon cette méthode, décrite plus précisément dans le document PCT/FR2007/000373, on détermine la présence de la séquence cible déterminée ainsi que sa quantité initiale lorsque le courant électrique diminue au fil des amplifications. En effet, durant le processus d'amplification le nombre de nucléotides libres des moyens d'amplification décroît puisqu'ils sont incorporés aux acides nucléiques synthétisés. L'agent oxydant ne peut alors réagir qu'avec une quantité de plus en plus limitée de nucléotides libres. En conséquence, de moins en moins de transferts électroniques dus à la réaction d'oxydation se produisent, et le courant électrique diminue.

Ainsi, une telle méthode permet de détecter et de quantifier rapidement la présence d'un acide nucléique donné dans un échantillon biologique quelconque.

Un problème qui se pose et que vise à résoudre la présente invention est non seulement de fournir une autre méthode de détection électrochimique qui permette de détecter la présence d'une séquence cible de nucléotides dans un échantillon biologique, mais aussi de pouvoir identifier sa nature et de la quantifier.

Dans le but de résoudre ce problème, selon un premier aspect, la présente invention propose une méthode d'identification électrochimique de séquences cibles de nucléotides. Ladite méthode étant du type selon laquelle on fournit un échantillon biologique susceptible de contenir une séquence cible de nucléotides déterminée et des moyens d'amplification activables comportant des nucléotides libres, pour provoquer la réplication de ladite séquence cible de nucléotides déterminée et la formation de séquences cibles de nucléotides répliquées. Selon la méthode on fournit également un composé oxydo-réductible apte à réagir vis-à-vis desdits nucléotides et on met en contact ledit composé oxydo-réductible avec ledit échantillon biologique. On active alors lesdits moyens d'amplification activables et on applique un champ électrique audit échantillon pour activer ledit composé oxydo-réductible et on mesure le courant électrique représentatif de l'activité électrochimique dudit composé oxydo-réductible, qui traverse ledit échantillon. Ainsi, on détermine la présence de ladite séquence cible de nucléotides déterminée si le courant électrique diminue. Selon l'invention, on fournit un composé oxydo-réductible apte à venir s'intercaler durant la réplication entre les nucléotides formant lesdites séquences cibles répliquées, lesdites séquences cibles répliquées provoquant l'inhibition de l'activité électrochimique dudit composé oxydo-réductible intercalé, par quoi le courant électrique diminue.

Outre les nucléotides libres, les moyens d'amplification activables comportent des amorces qui sont des acides oligonucléotidiques spécifiques et complémentaires de la séquence cible de nucléotides à détecter et une polymérase. Par ailleurs, ainsi qu'on l'expliquera ci-après de manière détaillée, le champ électrique est appliqué audit échantillon en mettant en contact des électrodes avec ledit échantillon et en appliquant une différence de potentiel entre ces électrodes.

Ainsi, une caractéristique de l'invention est de fournir un composé oxydo-réductible qui va non pas venir oxyder les bases nucléotidiques des nucléotides libres dans l'échantillon, comme cela est le cas dans le document de l'art antérieur précité, mais qui va venir s'intercaler entre les nucléotides formant les séquences cibles répliquées.

De manière préférentielle, on active lesdits moyens d'amplification activables selon des cycles d'amplification successifs et à chaque cycle d'amplification, on duplique ladite séquence cible répliquée. Le composé oxydo-réductible vient alors s'intercaler entre les nucléotides formant lesdites séquences cibles répliquées et perd son activité oxydo-réductible vis-à-vis du champ électrique appliqué. En conséquence au fil des cycles d'amplification le signal électrique mesuré diminue.

On enregistre alors avantageusement le nombre de cycle d'amplification correspondant à la diminution du courant électrique pour déterminer la concentration de ladite séquence cible de nucléotides dans ledit échantillon. En effet, la diminution du signal mesurée est proportionnelle à la quantité de séquence cible de nucléotides répliquée. De cette proportionnalité, il est possible de déduire la quantité de séquence cible de nucléotide déterminée présente initialement dans ledit échantillon. Ainsi, on répète l'activation desdits moyens d'amplification activables selon un certain nombre de cycles d'amplification, et on enregistre le nombre de cycle desdits moyens d'amplification lorsque le courant électrique diminue pour déterminer la concentration de ladite séquence cible de nucléotides dans ledit échantillon. En effet, plus l'échantillon biologique contient de séquences cibles de nucléotides déterminées, plus le nombre de cycles d'amplification nécessaires pour que l'intensité du courant électrique chute sera faible. Et à l'inverse, moins l'échantillon contient de séquences cibles déterminées, plus le nombre de cycles d'amplification sera grand. De la sorte, ainsi qu'on l'expliquera plus en détail dans la suite de la description, cette méthode permet également de quantifier la concentration en séquence cible déterminée à l'intérieur de l'échantillon biologique.

On précisera que « composé oxydo-réductible » qualifie non seulement les composé redox, mais aussi les composés aptes à s'oxyder dans certaines conditions, et à se réduire dans d'autres.

Selon un mode de réalisation de l'invention particulièrement avantageux, après que la séquence cible de nucléotides déterminée a été amplifiée, on fournit en outre de l'énergie thermique audit échantillon pour provoquer la libération dudit composé oxydo-réductible intercalé, et on applique un champ électrique audit échantillon pour enregistrer simultanément les variations de courant électrique qui traverse ledit échantillon. On détermine alors la quantité d'énergie thermique Q correspondant aux variations maximales du courant électrique enregistrées pour identifier la nature de ladite séquence cible de nucléotides déterminée. Avantageusement, on fournit de l'énergie thermique audit échantillon de manière à provoquer une augmentation progressive de la température dudit échantillon. Ainsi, on enregistre les variations de courant électrique en fonction de l'énergie thermique fournie, ou plus concrètement de la température sur une gamme donnée. De la variation maximale du courant électrique à une température donnée, on identifie la nature de ladite séquence cible de nucléotides déterminée. En effet, selon la nature de la séquence cible amplifiée, la variation maximale du courant électrique à une température donnée est caractéristique de ladite séquence cible de nucléotides répliquée.

Ainsi, lorsque l'on fournit suffisamment d'énergie thermique à l'échantillon et par conséquent aux séquences cibles de nucléotides répliquées, les doubles brins des séquences cibles de nucléotides répliquées tendent à se séparer en deux monobrins et à libérer en conséquence le composé oxydo-réductible intercalé qui retrouve alors son activité électrochimique. Les doubles brins des séquences cibles de nucléotides répliquées se séparent les uns des autres pour une agitation thermique donnée, c'est-à-dire à une température donnée de telle sorte que la libération du composé oxydo-réductible, qui est alors mesurée par l'intermédiaire du courant électrique généré aux électrodes, intervient brusquement, d'une manière quasiment discrète, c'est à dire sur une gamme de température étroite, lorsqu'une quantité déterminée d'énergie thermique est cédée à l'échantillon.

Avantageusement, on fournit de l'énergie thermique audit échantillon de manière à provoquer une augmentation progressive de la température dudit échantillon, par exemple entre 40°C et 98°C. Dans cette gamme de températures, les doubles brins de la séquence cible de nucléotides répliquée vont progressivement évoluer d'un état apparié ou chacune des bases nucléotidiques des séquences cibles répliquées sont associées de manière complémentaire vers un état dissociée où chaque séquence cible répliquée finira par être monobrin. Le passage d'un état double brin à un état monobrin s'effectue donc dans une gamme étroite de température, se caractérisant par une température dite de dissociation, généralement notée Tm, caractéristique de la nature de la séquence cible de nucléotides répliquée.

En outre, on mesure préférentiellement le courant électrique représentatif de l'activité électrochimique dudit composé oxydo-réductible à une température prédéterminée dudit échantillon, à laquelle aucune autre molécule formée ou en cours de formation, n'inhibe le composé oxydo-réductible, par exemple les dimères d'amorce. Avantageusement, ladite température prédéterminée est sensiblement inférieure, mais néanmoins proche de la température dudit échantillon correspondant à la quantité d'énergie thermique Q déterminée, de sorte qu'à cette température élevée, toutes les autres molécules, d'une taille plus modeste que la séquence cible de nucléotides répliquée, sont déshybridées, tandis que la séquence cible de nucléotides répliquée demeure intacte. De la sorte, on élimine du courant électrique mesuré, la quantité de courant issue de la libération dudit composé oxydo-réductible par les divers dimères d'amorces ou de toutes autres molécules plus petites, que l'énergie thermique déjà fournie a permis de dissocier.

De manière préférée, on utilise la voltampérométrie pour mesurer et enregistrer le courant électrique ou lesdites variations de courant électrique. Cette méthode potentiodynamique consiste à appliquer entre deux électrodes, ainsi qu'on l'expliquera plus en détail dans la suite de la description, un potentiel variable dans le temps et à enregistrer concomitamment la variation de courant qui en résulte. Préférentiellement, on utilise la voltampérométrie à vague carrée. D'autre techniques électrochimiques sont bien évidemment potentiellement applicables, la voltampérométrie à impulsion différentielle par exemple ou la voltampérométrie à balayage linéaire ou cyclique ou encore la voltampérométrie à courant échantillonné et la voltampérométrie à courant alternatif.

Selon un mode de mise en oeuvre de l'invention particulièrement avantageux, ledit composé oxydo-réductible fourni est un complexe d'un métal de transition, par exemple un métal de la colonne VIIIA du tableau de Mendeleïev, et plus précisément l'osmium. En outre, le composé oxydo-réductible présente avantageusement au moins un ligand intercalant de séquence d'acide nucléique et par exemple un ligand dipyridophenazine, lequel est adapté à venir s'intercaler entre les brins appariés formés par les séquences cibles de nucléotides répliquées.

Au surplus, le composé oxydo-réductible présente au moins un ligand bipyridine et avantageusement deux ligands de ce type. On décrira plus en détail dans la suite de la description un complexe de l'osmium assorti d'un ligand dipyridophenazine et de deux ligands bipyridine. On observera par ailleurs, que certains composés oxydo-réductibles entièrement organiques peuvent également être mis en oeuvre avec profit, c'est par exemple le cas du bleu de méthylène.

Selon un mode de mise en oeuvre de l'invention préféré, on provoque la réplication de ladite séquence cible de nucléotides déterminée et la formation de séquences cibles de nucléotides répliquées sous la forme de double brins d'acides nucléiques. Aussi, lesdits moyens d'amplification activables sont de type PCR.

Selon un second aspect, la présente invention propose un ensemble d'identification électrochimique de séquences cibles de nucléotides. Ledit ensemble comprend des moyens pour recevoir un échantillon biologique susceptible de contenir une séquence cible de nucléotides déterminée et des moyens d'amplification activables comportant des nucléotides libres, pour provoquer la réplication de ladite séquence cible de nucléotides déterminée et la formation de séquences cibles de nucléotides répliquées. En outre, l'ensemble inclut un composé oxydo-réductible apte à réagir vis-à-vis desdits nucléotides, ledit composé oxydo-réductible étant mis en contact avec ledit échantillon biologique. Des moyens d'activation permettent d'activer lesdits moyens d'amplification activables, et des moyens permettent d'appliquer un champ électrique audit échantillon de façon à activer ledit composé oxydo-réductible, tandis que des moyens de mesure permettent de mesurer le courant électrique représentatif de l'activité électrochimique dudit composé oxydo-réductible, qui traverse ledit échantillon. Enfin, des moyens de détermination permettent de déterminer la présence de ladite séquence cible de nucléotides déterminée si le courant électrique diminue. Selon l'invention, ledit composé oxydo-réductible est choisi parmi les composés aptes à venir s'intercaler durant la réplication entre les nucléotides formant lesdites séquences cibles répliquées, soit dans les doubles brins de la séquence cible de nucléotides répliquée, lesdites séquences cibles répliquées provoquant l'inhibition de l'activité électrochimique dudit composé oxydo-réductible intercalé, par quoi le courant électrique diminue.

En outre, et selon un mode de mise en oeuvre avantageux, l'ensemble d'identification comprend en outre des moyens pour fournir de l'énergie thermique audit échantillon de façon à provoquer la libération dudit composé oxydo-réductible intercalé, et des moyens pour appliquer un champ électrique audit échantillon, grâce à des électrodes mises en contact avec l'échantillon, de manière à enregistrer simultanément, en fonction de la température, les variations de courant électrique qui traverse ledit échantillon ; et aussi, des moyens pour déterminer la quantité d'énergie thermique correspondant aux variations maximales du courant électrique enregistrées pour contrôler la présence de ladite séquence cible de nucléotides déterminée. Ces moyens déterminent à partir de la variation maximale du courant électrique enregistrée la température de dissociation (Tm) caractéristique de la présence de séquence cible de nucléotides à détecter. On expliquera plus en détail dans la description qui va suivre, l'ensemble d'identification électrochimique précité qui permet de mettre en oeuvre la méthode conforme à l'invention. En particulier, on décrira les moyens d'activation, lesquels sont avantageusement adaptés à activer lesdits moyens d'amplification activables selon des cycles d'amplification successifs pour dupliquer ladite séquence cible répliquée à chaque cycle d'amplification. En outre, l'ensemble d'identification selon l'invention, comprend des moyens d'enregistrement pour enregistrer le nombre de cycle d'amplification correspondant à la diminution du courant électrique de manière à déterminer la concentration de ladite séquence cible de nucléotides dans ledit échantillon.

De plus, d'autres particularités et avantages de l'invention ressortiront à la lecture de la description faite ci-après d'un mode de réalisation particulier de l'invention, donné à titre indicatif mais non limitatif, en référence aux dessins annexés sur lesquels :
- la Figure 1 est une vue schématique d'un ensemble d'identification électrochimique conforme à l'invention;
- les Figures 2A à 2C sont des vues schématiques d'un élément de l'ensemble d'identification représenté sur la Figure 1, selon une variante d'exécution ;
- les Figures 3A et 3B sont des diagrammes intensité/potentiel obtenus grâce à l'ensemble d'identification représenté sur la Figure 1 ;
- la Figure 3C est une vue d'un diagramme obtenus par déduction des diagrammes représentés sur les Figures 3A et 3B ;
- la Figure 4 est une vue d'un diagramme d'un type analogue à celui de la Figure 3C ;
- la Figure 5A illustre un diagramme intensité/température obtenu grâce à l'ensemble d'identification représenté sur la Figure 1 et dans une première étape ;
- la Figure 5B illustre un diagramme obtenu dans une seconde étape par transposition du diagramme illustré sur la Figure 5A ;
- la Figure 6 illustre un diagramme du type représenté sur la Figure 5B, dans une application particulière ;

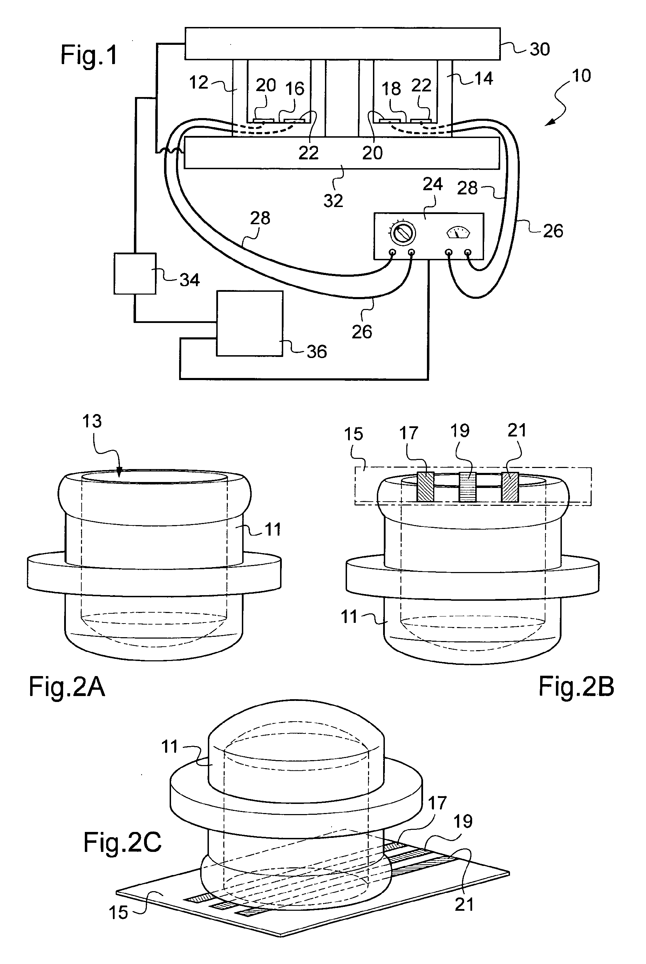

La méthode d'identification électrochimique conforme à l'invention nécessite la mise en oeuvre d'un ensemble d'identification électrochimique comportant d'une part des moyens de contrôle et de mesure que l'on décrira dans un premier temps et d'autre part, des constituants biologiques et chimiques spécifiques. Ainsi, une installation d'identification électrochimique 10 conforme à l'invention a été représentée de manière schématique sur la Figure 1. Cette Figure 1 montre deux micro-cuvettes 12, 14, adaptées à recevoir à l'intérieur, un échantillon biologique dont on définira ci-après les caractéristiques. Ces micro-cuvettes 12, 14, déjà connues selon l'art antérieur, présentent respectivement un fond 16, 18, sur lequel sont présentes, une électrode 20, une contre-électrode 22, et une électrode de référence non représentée, obtenues par exemple par sérigraphie et respectivement reliées à un potentiostat 24, les deux premières par l'intermédiaire de fils conducteurs 26, 28. En outre, elles sont prises en sandwich entre un module à effet Pelletier 32 qui les supporte et un couvercle chauffant 30, reliés à un générateur 34. Ainsi qu'on l'expliquera ci-après, le module à effet Pelletier 32, permet de céder une quantité d'énergie thermique donnée au contenu des micro-cuvettes 12, 14. Par ailleurs, le générateur 34 et le potentiostat 24 sont contrôlés par un micro-ordinateur 36, lequel contient un programme d'ordinateur et des moyens d'enregistrement.

Ainsi, ces micro-cuvettes 12, 14, constituent des moyens de réception apte à recevoir un échantillon biologique, lequel échantillon biologique est susceptible d'inclure une séquence d'acide nucléique, et notamment de l'ADN, lequel renferme une séquence cible de nucléotides déterminée que l'on cherche à détecter. En outre, le module à effet Pelletier 32, et le générateur 34 apte à être piloté par l'intermédiaire du micro-ordinateur 36, constituent une première partie de moyens d'amplification activables, la seconde partie étant constituée par du matériel biologique et des composés chimiques.

Bien évidemment, d'autres moyens de réception bien connus, non représentés, comprennent un tube dans le culot duquel on vient porter l'échantillon biologique. Le tube est alors équipé d'électrodes aptes à plonger dans cet échantillon biologique et à être reliées au potentiostat. Le tube est alors destiné à être installé dans un thermocycleur pour porter les échantillons biologiques à des températures prédéterminées et selon des cycles de temps prédéfinis. D'autres moyens de réception encore, illustrés sur les Figures 2A à 2C, permettent de recevoir les échantillons biologiques. Ils comprennent un récipient 11 de faible dimension, par exemple un bouchon de tube, représenté sur la Figure 2A, autorisant une contenance de 1µL à 1 mL par exemple, et ouvert dans la partie supérieure 13. Il est susceptible de recevoir le mélange réactionnel. Il est alors refermé hermétiquement au moyen d'un film 15 sur lequel ont été sérigraphiées trois électrodes disjointes 17, 19, 21. Les électrodes 17, 19, 21 sont orientées vers l'intérieur du récipient 11 et elles en ressortent à la jointure entre le bord du récipient et le film 15. Ensuite, le récipient 11 muni de son film 15 est alors retourné pour être porté en appui sur un module à effet Pelletier et partant, le mélange réactionnel initialement dans le fond du récipient 11 vient en contact avec les électrodes 17, 19, 21, qu'il baigne.

La méthode d'amplification ici utilisée est la méthode dite PCR. Aussi, grâce au module à effet Pelletier 32, l'intérieur des micro-cuvettes 12, 14, est adapté à être porté à des températures déterminées pendant des temps déterminés également et selon le protocole suivant : le protocole démarre avec un palier préliminaire de 1 à 15 minutes suivant le type de polymérase où l'intérieur des micro-cuvettes 12, 14, est porté à une température de 94-95° C ; ensuite, un certain nombre de cycles consécutifs de température en fonction de l'amplification nécessaire à la détection de la séquence cible de nucléotides répliquée, classiquement entre 10 et 50 cycles, sont imposés aux micro-cuvettes 12, 14, selon quatre paliers successifs par cycle, un premier palier dit de dénaturation classiquement de 1 à 60 secondes à une température de 94-95° C ; un deuxième palier dit d'appariement des amorces, classiquement de 1 à 60 secondes à une température comprise entre 40°C et 72°C, caractéristique des amorces spécifiques de la séquence cible de nucléotides déterminée ; un troisième palier dit d'élongation, classiquement de 1 à 60 secondes à 72°C et un dernier palier de quelques secondes à une température comprise entre 40°C et 95°C, laquelle est déterminée par le temps nécessaire à la mesure électrochimique. On expliquera ci-après les conditions de mise en oeuvre de cette première partie de moyens d'amplification après avoir décrit la seconde partie comprenant notamment du matériel biologique et des composés chimiques.

Un des objets de l'invention étant d'amplifier une séquence d'acide nucléique par réplication et de mesurer électrochimiquement sa présence dans le milieu au cours du processus d'amplification, il convient de disposer tout d'abord de matériel biologique permettant cette amplification. Afin de mettre en oeuvre la technique de PCR, il convient de mettre en contact avec l'acide nucléique à amplifier, une enzyme polymérase, une paire d'amorces («primer » en langue anglaise) et les quatre nucléotides libres : dGTP, dATP, dTTP et dCTP constituant de l'ADN, respectivement désoxy-guanine-tri-phosphate, désoxy-adénosine-tri-phosphate, désoxy-thymidine-tri-phosphate et désoxy-cytosine-tri-phosphate. Des analogues de bases du type désoxyribonucléotide-triphosphates telles que la désoxy-déazaguanine-triphosphate, peuvent également être employées.

Ainsi, sont introduits dans l'une des micro-cuvettes 12, et selon un premier exemple d'application, outre un échantillon biologique à tester et précisément un extrait d'ADN de cytomégalovirus contenant une séquence cible d'acides nucléiques de 283 paires de bases, l'ADN polymérase c'est-à-dire l'enzyme, les amorces, et les quatre types de nucléotides, et le tout formant un mélange réactionnel qui est bien évidemment liquide et tamponné. En outre, afin de pouvoir mesurer l'intensité d'un signal électrique, on ajoute au mélange réactionnel un composé oxydo-réductible, et en l'espèce le complexe de l'osmium : bis(2,2'-bipyridine)dipyrido[3,2-a :2',3'-c]phenazine osmium (II) dont le numéro CAS peut-être 3555395-37-8 noté dans notre cas [Os^{II}(bpy)₂DPPZ]²⁺ et de formule I :

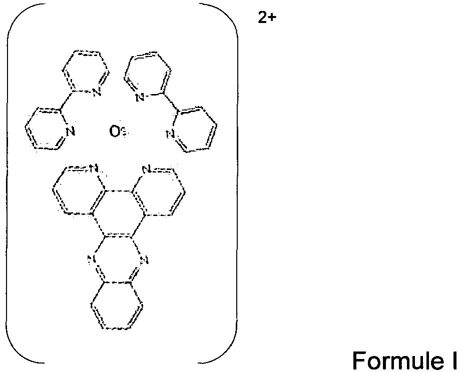

D'autres composés oxydo-réductible sont envisageables, et notamment avec le ruthénium à la place de l'osmium. D'autres ligands sont aussi susceptibles d'être mis en oeuvre. L'avantage du ligand dipyridophenazine réside dans sa capacité à venir s'intercaler entre les nucléotides formant les séquences cibles de l'acide nucléique. On citera par exemple, comme autres ligands envisageables, le DPPX : 7,8-dimethyl-dipyrido[3,2-a:2_,3_- c]phenazine ; le PTDB : 3-(pyridine-2-yl)-5,6-diphenyl-as-triazine ; ou le DPT : 3-(pyrazin-2yl)-*as*-triazino[5,6-*f*]phenanthrene ; ou encore les ligands présentant une fonction quinone, tel le PHI : phenanthrène quinone diimine.

Des composés organiques intercalant de l'ADN et oxydo-réductibles sont également susceptibles d'être mis en oeuvre dans la méthode objet de l'invention. On citera par exemple, le bromure d'éthidium, l'acridine et ses dérivés, les dérivés de l'acridone ou encore les dérivés de la phénazine.

À des fins d'expérimentation et de contrôle, sont introduits dans l'autre micro-cuvette 14, les éléments identiques précités, excepté l'échantillon biologique à tester. Selon un exemple de mise en oeuvre, les concentrations des différents éléments introduits dans les micro-cuvettes 12, 14, sont reportées dans le tableau I ci-dessous.

**Tableau I**

| | Micro-cuvette 12 | Micro-cuvette 14 |
|---|---|---|
| Amorces gauches | 250 nM | |
| Amorces droites | 250 nM | |
| Nucléotides libres :dnTP | 200 µM | |
| Tampon PCR | 1 x | |
| Enzyme :Hot Star Taq | 0,1 U/µL | |
| [Os(bpy)₂-dppz] | 0,5 µM | |
| Extrait d'ADN de cytomegalovirus de 283 paires de bases | 100 000 copies | 0 copies |

Ainsi, le mélange réactionnel contenu dans les deux micro-cuvettes 12, 14, est porté grâce au module à effet Pelletier 32, piloté par le micro-ordinateur 36, à différentes températures durant des périodes déterminées. Après le palier préliminaire où le mélange réactionnel est porté une seule fois à une température de 95° C pendant 15 minutes, le mélange réactionnel est porté sur le premier palier, à une température de 94° C pendant 30 secondes de manière à déshybrider les séquences cibles des acides nucléiques, c'est-à-dire à dissocier les deux brins complémentaires des séquences cibles des acides nucléiques ; ensuite il est porté sur le deuxième palier à 53° C durant 60 secondes afin que les amorces respectives viennent s'hybrider sur les brins d'ADN dissociés ; puis il est porté sur le troisième palier à 72°C durant 60 secondes pour permettre aux polymérases de synthétiser un brin complémentaire et former ainsi l'amplicon et la séquence cible répliquée qu'il inclut. Enfin, le mélange réactionnel est porté à une température de 85° C, sur un quatrième palier durant 10 secondes pendant lesquelles on commande la mise en oeuvre du potentiostat 24 par l'intermédiaire du micro-ordinateur 36.

Selon le premier exemple d'application défini ci-dessus, sur le quatrième palier prédéfini et durant la période de 10 secondes, grâce au potentiostat 24, on enregistre par voltampérométrie à vagues carrées, la courbe intensité/potentiel dans une zone de potentiel encadrant le potentiel standard du composé oxydo-réductible. On impose ainsi une différence de potentiel entre l'électrode 20 et la contre-électrode 22 et ont fait varier cette différence de potentiel selon un profil à vagues carrées. Parallèlement, on mesure le courant électrique qui traverse ces électrodes et on obtient alors une courbe intensité/potentiel en forme de pic dont la valeur de courant maximale, après soustraction d'une ligne de base, est représentative de la concentration en composé oxydo-réductible non intercalé, présent en solution.

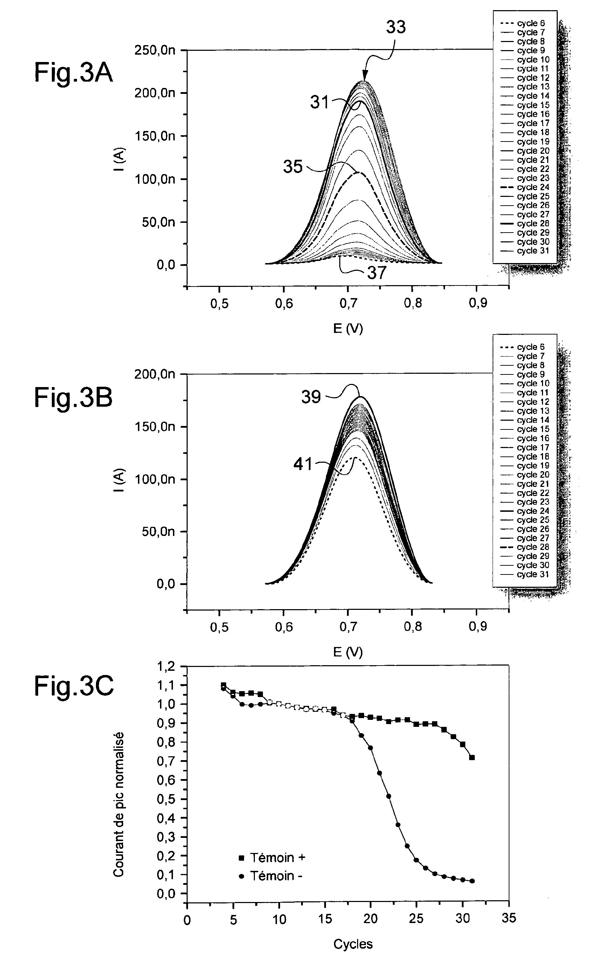

On se reportera ainsi sur les Figures 3A à 3B tout d'abord, puis sur la Figure 3C. Précisément, 31 cycles de réplication ont été imposés aux échantillons biologiques contenus dans les micro-cuvettes 12, 14. La Figure 3A illustre l'évolution des courbes intensité/potentiel en fonction des cycles, pour les échantillons biologiques incluant la séquence cible de nucléotides recherchée, tandis que la Figure 3B représente l'évolution des courbes intensité/potentiel en fonction des cycles, pour le mélange réactionnel dénué de séquence cible. Sur la Figure 3A, c'est à partir du 18e cycle 31 que le sommet 33 de la courbe s'abaisse significativement c'est-à-dire que la consommation en composé oxydo-réductible est sensible, pour atteindre la mi-hauteur 35 au 22^{e} cycle. Au 31^{e} cycle, la courbe 37 devient pratiquement plane et son sommet atteint une valeur d'intensité inférieure à 5% à celles des premiers cycles. Ainsi, le composé oxydo-réductible a été incorporé à l'intérieur des molécules double brin d'ADN formées. Cela, implique, que la séquence cible de nucléotides recherchée était bien présente dans les échantillons biologiques.

A l'inverse, sur la Figure 3B, l'extremum 39 de la courbe demeure à une valeur sensiblement constante jusqu'au 31^{e} cycle, en comparaison de la courbe précédente, car dans ces échantillons la séquence cible de nucléotides recherchée n'est pas présente. Néanmoins, il diminue légèrement notamment à cause de la formation de dimères d'amorce dans le mélange réactionnel.

Ainsi, on détecte rapidement la présence d'une séquence cible de nucléotides recherchée, dans un échantillon biologique quelconque au moyen du procédé décrit ci-dessus.

On observera que la valeur du courant de pic est normalisée, en la divisant par la valeur moyenne corrigée de la dérive des courants de pic obtenus pendant les premiers cycles d'amplification, c'est-à-dire lorsque la quantité de séquence cible de nucléotides répliquée n'est pas encore suffisante pour faire chuter significativement le courant de pic mesuré, par exemple au 5^{e} cycle.

On se reportera sur la Figure 3C illustrant les courbes tirées des deux séries de courbes précitées et montrant la valeur du courant de pic ainsi normalisée en fonction du nombre de cycles réalisés et ce, pour les deux micro-cuvettes 12, 14, comportant respectivement l'échantillon biologique à tester et le matériel biologique destiné à la réplication ainsi que le composé oxydo-réductible et l'autre, le seul matériel biologique avec le composé oxydo-réductible.

Ainsi, on observe que, jusqu'au 25^{e} cycle, la valeur du courant qui traverse les mélanges réactionnels des deux micro-cuvettes 12, 14, est sensiblement parallèle et relativement constante. En revanche, entre le 25^{e} cycle, et au-delà du 30^{e} cycle, on observe que la valeur du courant électrique qui traverse la micro-cuvette 12 incluant l'échantillon biologique à tester chute brutalement en comparaison du courant électrique qui traverse l'autre micro-cuvette 14, dénuée elle, de la séquence cible d'acides nucléiques déterminée. Cette chute brutale et exponentielle du courant électrique, selon une fonction en ε^{C}, c étant le nombre de cycles et ε l'intensité d'amplification proche de 2, témoigne de l'amplification de la séquence cible de nucléotides déterminée, dont les amorces étaient spécifiques, et de la production en conséquence de séquences cibles répliquées. En effet, la production de molécules de séquences cibles d'acide nucléiques répliquées provoque alors l'inactivation du composé oxydo-réductible précité, via l'intercalation de celui-ci dans le double brin formé de la séquence d'acide nucléique répliquée. Ainsi inactif, le composé oxydo-réductible ne peut plus échanger de charges avec la surface des électrodes 20, 22, et par conséquence ne plus être détecter électrochimiquement. Cela se traduit par une chute du signal électrique qui est alors révélatrice de la formation de la séquence cible de nucléotides déterminée par l'intermédiaire des amorces spécifiques, dans l'échantillon biologique exploré.

L'enregistrement du courant est réalisé à une température supérieure à la température dite de dissociation des dimères d'amorces et/ou autres séquences d'ADN non spécifiquement amplifiées. Le choix de la température à laquelle on effectue la mesure est un paramètre critique pour discriminer entre un échantillon faux positif et un vrai positif.

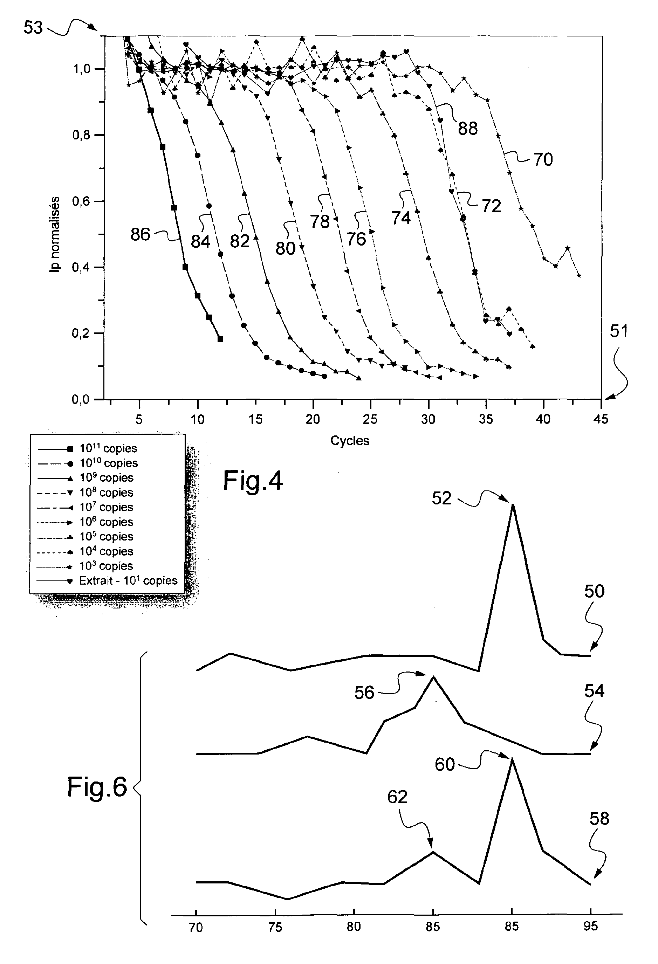

Par ailleurs, on se référera à présent au graphique illustré sur la Figure 4, pour décrire, selon un deuxième exemple d'application, le principe de la quantification d'une séquence cible de nucléotides déterminée dans un échantillon donné.

Sur l'axe des abscisses 51 du graphique, sont portés les nombres de cycles de réplication des moyens d'amplification, et sur l'axe des ordonnées 53 sont reportées les valeurs normalisées du courant maximal relevées à chaque cycle suivant le mode de mise en oeuvre illustré sur la Figure 3A.

Sur ce graphique de la Figure 4, neuf courbes d'étalonnage sont représentées de droite à gauche, 70, 72, 74, 76, 78, 80, 82, 84, 86, et elles présentent des portions intermédiaires sensiblement parallèles entre elles et selon une direction proche de la verticale. Ces courbes correspondent respectivement aux différents tracés obtenus en partant d'un échantillon biologique comprenant 10³ copies de la séquence cible contenue dans le génome du Cytomégalovirus pour la première 70 des neuf courbes, et 10¹¹ pour la neuvième 86. De la première courbe 70 à la dernière 86, le nombre de copies est pour chaque courbe suivante, est multiplié par dix.

Ainsi, on constate que, plus l'échantillon biologique contient une quantité importante de séquence cible de nucléotides déterminée, plus le courant électrique qui traverse l'électrode diminue tôt en fonction du nombre de cycles. En effet, plus l'échantillon contient d'acides nucléiques à l'origine, incorporant la séquence cible déterminée, et moins grand est le nombre de cycles nécessaires pour produire par réplication une même quantité de séquences cibles de nucléotides déterminée, et par conséquent plus tôt est la diminution du courant via l'intercalation du composé oxydo-réductible dans les doubles brins des séquences cibles de nucléotides répliquées. De la sorte, on comprend qu'une mesure de la quantité d'acides nucléiques incorporant la séquence cible est possible, en déterminant le nombre de cycles à partir duquel, la quantité de courant qui traverse l'échantillon s'infléchit. En outre, la première courbe 70 sur la Figure 4 montre que la présence de la séquence cible est encore détectable lorsque seulement 1000 copies de ladite séquence cible sont initialement présentes dans l'échantillon.

Un extrait initialement de concentration en séquence cible d'acides nucléiques déterminée a ainsi été testé, et sa courbe 88 apparaît en traits interrompus le long de la deuxième courbe 72 d'étalonnage correspondant à 10⁴ copies de la séquence cible.

Ainsi donc, la méthode selon l'invention appliquée à un échantillon biologique susceptible de contenir une séquence cible de nucléotides déterminée permet non seulement, grâce à la mise en oeuvre d'une méthode d'amplification et d'une mesure électrochimique d'un agent oxydo-réductible intercalant de l'ADN double brin de révéler la présence ou l'absence de la séquence cible de nucléotides déterminée, mais également de le quantifier avec une amplitude du signal, une reproductibilité et une sensibilité améliorées en comparaison des autres méthodes électrochimiques selon l'art antérieur. Cette méthode tire partie des propriétés oxydo-réductible d'un agent intercalant de séquences d'acide nucléiques formant un double brin qui n'est nullement mis en oeuvre dans les méthodes d'amplification classiques, pour révéler la présence d'une séquence cible d'acide nucléique.

Dans le but d'affiner la méthode de détection, notamment en terme de spécificité d'identification de la séquence cible amplifiée, on provoque, à l'issue de l'amplification, la déshybridation progressive de toutes les séquences cibles répliquées à travers une montée progressive de l'échantillon en température, de manière à libérer le composé oxydo-réductible intercalé. Redevenant alors électrochimiquement détectable, ce composé oxydo-réductible est de nouveau apte à fournir un signal électrique aux électrodes, représentatif de la quantité de composé oxydo-réductible libéré et par conséquent, de la nature et donc de la longueur de la séquence cible de nucléotides déterminée.

Cette déshybridation est réalisée en portant progressivement, selon une rampe de température appropriée, les molécules d'ADN d'une température ou tous les doubles brins sont appariés, c'est-à-dire vers 40 °C, jusqu'à une température ou tous les doubles brins sont dissociés, c'est-à-dire vers 98 °C, et le module à effet Pelletier 32 précité constitue un moyen privilégié pour cela. Ces derniers permettent en effet de fournir de l'énergie thermique au mélange réactionnel contenu dans les deux micro-cuvettes 12, 14, représentées sur la Figure 1, de façon à provoquer la déshybridation des séquences cible de nucléotides répliquées et partant, la libération dudit composé oxydo-réductible intercalé. En outre, grâce au potentiostat 24 et par l'intermédiaire du micro-ordinateur 36, on applique alors une différence de potentiel entre l'électrode 20 et la contre-électrode 22 et on fait varier cette différence de potentiel selon le profil à vagues carrées précité. On mesure le courant électrique qui traverse ces électrodes et on détermine de la sorte, un courant de pic.

Ainsi, grâce au module à effet Pelletier 32, on incrémente la température des mélanges réactionnels, par exemple degré par degré, entre 70° C et 95° C. Parallèlement, dès que la température des mélanges réactionnels augmente d'1 °C, on impose une différence de potentiel entre les électrodes 20, 22, et on mesure le courant qui les traverse, selon la méthode voltampérométrique précitée.

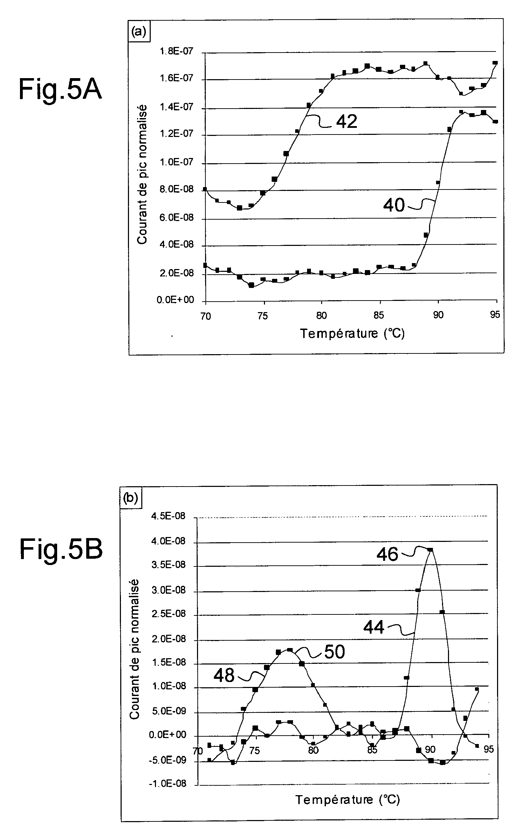

On se reportera à présent sur la Figure 5A montrant, selon un troisième exemple d'application, un diagramme du courant de pic alors obtenu en fonction de la température, et ce pour les deux micro-cuvettes 12, 14, l'une incluant le mélange réactionnel avec la séquence cible de nucléotides recherchée, l'autre, le mélange réactionnel sans cette séquence cible.

La courbe inférieure 40 ainsi obtenue correspond au mélange réactionnel incluant la séquence cible de nucléotides et par conséquent une pluralité de séquences cibles de nucléotides répliquées. Ainsi, on observe que l'augmentation de la température du mélange réactionnel, entre 70° C et 88° C, ne produit aucun effet sur les molécules d'ADN incluant les séquences cibles de nucléotides. En revanche, entre 88°C et 92°C, le courant de pic est multiplié par sept. Ce courant de pic est alors directement proportionnel à la quantité de composé oxydo-réductible libéré et par conséquent, de la nature et donc de la longueur de la séquence cible d'acide nucléique déterminée qui est ici de 283 paires de bases.

On observera avec la courbe supérieure 42, correspondant au mélange réactionnel dénué de séquence cible d'acide nucléique déterminée, que le signal électrique mesuré double entre 70°C et 85°C. Cela est la conséquence de l'intercalation dans les dimères d'amorce et autres doubles brins synthétisés de façon non spécifique et de taille inférieure à la séquence cible d'acide nucléique déterminée.

La mise en lumière de la séquence cible de nucléotides recherchée, est alors plus probante en transposant chaque point des courbes représentées sur la Figure 5A, en la valeur de la dérivée en ce point. On obtient de la sorte les courbes de fusion représentées sur la Figure 5B.

Ainsi, la courbe inférieure 40 correspondant au mélange réactionnel incluant la séquence cible, est-elle transformée en une courbe typique 44 présentant un pic significatif 46 à la température de 90°C. Bien évidemment, ce pic significatif 46 correspond à la brusque variation de courant de pic observée sur la Figure 5A pour la courbe inférieure 40. Ce pic significatif 46 est représentatif de la nature et donc de la longueur de la séquence cible déterminée par la température à laquelle il apparaît. En effet, plus la séquence cible recherchée est principalement longue, plus la quantité de composé oxydo-réductible incluse dans les amplicons est importante, bien évidemment pour une quantité équivalente d'amplicons. Aussi, pour libérer les molécules du composé oxydo-réductible il sera nécessaire d'apporter une plus grande quantité d'énergie thermique afin de déshybrider le double brin formé par la séquence cible déterminée. Par conséquent, la température à laquelle cette déshybridation interviendra sera d'autant plus importante. Au surplus, la quantité de molécules de composé oxydo-réductible incluse dans les amplicons étant plus importante, une fois libérée, le signal électrique recueilli sera également d'autant plus important.

En conséquence, plus la séquence cible de nucléotides recherchée est de nature principalement longue, plus le pic significatif 46 est important et décalé vers une température élevée.

En revanche, s'agissant de la courbe supérieure 42 représentée sur la Figure 5A, sa transformation en courbe dérivée 48 représentée sur la Figure 5B, fait apparaître un pic élargi 50 à une température comprise entre 75°C et 80°C. Ce pic élargi 50 correspond tout simplement à la déshybridation des dimères d'amorces et autres doubles brins synthétisés de façon non spécifique et de taille inférieure à la séquence cible d'acide nucléique déterminée, qui provoque alors la libération du composé oxydo-réductible. On observera que ce pic élargi 50, apparaît dans une gamme de températures inférieure à celle du pic significatif 46, et qu'il est plus diffus.

Selon un quatrième exemple d'application, on montre que l'on peut détecter la présence d'au moins deux séquences cibles de nucléotides distinctes, dans un même échantillon biologique, grâce à la méthode d'identification conforme à l'invention.

Pour ce faire, on utilise bien évidemment l'ensemble d'identification représenté sur la Figure 1, et on effectue trois séries de mesures conformément aux exemples d'application décrits ci-dessus. Il s'agit ici de montrer que l'on peut identifier dans un même échantillon biologique, la présence d'une bactérie, Achromobacter Xylosoxidans, dont la taille de la séquence cible de nucléotides est de 100 paires de bases et le Cytomégalovirus humain, précédemment utilisé, dont la taille de la séquence cible de nucléotides est de 283 paires de bases. Une première série de mesures correspond au Cytomégalovirus humain et est réalisée dans les mêmes conditions que celles décrites ci-dessus. Une deuxième série de mesures correspond précisément à la bactérie Achromobacter Xylosoxidans et en conséquence, le matériel d'amplification inclut les amorces spécifiques de la séquence cible correspondante. Et une troisième série de mesures correspond au mélange du Cytomégalovirus humain et de la bactérie Achromobacter Xylosoxidans, et par conséquent le matériel d'amplification inclut dans ce cas, les deux amorces spécifiques correspondantes dans le mélange.

On réalise alors conformément au troisième exemple d'application, les courbes de fusion pour les trois séries de mesures.

On a représenté sur la Figure 6, les trois courbes alors obtenues et pour une plus grande clarté elles ont été décalées selon l'axe des ordonnées les unes par rapport aux autres. Ainsi, on retrouve sur cette Figure 6, une première courbe 50, concernant le Cytomégalovirus humain, et qui correspond à la courbe typique 44 illustrée sur la Figure 5B. On retrouve ainsi un premier pic significatif 52 pour une valeur de température équivalente à 90° C. Par ailleurs, s'agissant de la bactérie Achromobacter Xylosoxidans, la courbe de fusion 54 correspondant à la deuxième série de mesures se caractérise par un deuxième pic significatif 56 pour une valeur de température voisine de 85° C.

Et enfin, la troisième série de mesures conduit à une troisième courbe 58 présentant un troisième 60 et un quatrième 62 pics significatifs respectivement pour des valeurs de température équivalente à 90° C et à 85° C. Ces valeurs correspondent exactement à celles de la bactérie seule et du Cytomégalovirus humain seul.

On montre ainsi, par l'intermédiaire de ce quatrième exemple d'application, la possibilité grâce à la méthode d'identification conforme à l'invention, de détecter une pluralité de séquence cible de nucléotides dans un même échantillon biologique.

Selon un mode de mise en oeuvre de l'invention particulièrement avantageux, et non représenté ici, mais conformément à l'invention, il est prévu de pouvoir amplifier une séquence cible possédant ou non certaines différences dans sa séquence et d'identifier la présence ou non de ces différences par une courbe de fusion, telle que définie ci-dessus.

## Revendications

1. Méthode d'identification électrochimique de séquences cibles de nucléotides, ladite méthode étant du type selon laquelle :
- on fournit un échantillon biologique susceptible de contenir une séquence cible de nucléotides déterminée ;
- on fournit des moyens d'amplification activables comportant des nucléotides libres, pour provoquer la réplication de ladite séquence cible de nucléotides déterminée et la formation de séquences cibles de nucléotides répliquées sous la forme de double brins d'acides nucléiques ;
- on fournit un composé oxydo-réductible apte à réagir vis-à-vis desdits nucléotides et on met en contact ledit composé oxydo-réductible avec ledit échantillon biologique ;
- on active lesdits moyens d'amplification activables ;
- on applique un champ électrique audit échantillon pour activer ledit composé oxydo-réductible et on mesure le courant électrique représentatif de l'activité électrochimique dudit composé oxydo-réductible, qui traverse ledit échantillon ; et,
- on détermine la présence de ladite séquence cible de nucléotides déterminée si le courant électrique diminue ;
**caractérisée en ce qu'**on fournit un composé oxydo-réductible venant s'intercaler durant la réplication entre les nucléotides formant lesdites séquences cibles répliquées, lesdites séquences cibles répliquées provoquant l'inhibition de l'activité électrochimique dudit composé oxydo-réductible intercalé, par quoi le courant électrique diminue.

2. Méthode d'identification selon la revendication 1, **caractérisée en ce qu'**on active lesdits moyens d'amplification activables selon des cycles d'amplification successifs et **en ce qu'**à chaque cycle d'amplification on duplique ladite séquence cible répliquée.

3. Méthode d'identification selon la revendication 2, **caractérisée en ce qu'**on enregistre le nombre de cycle d'amplification correspondant à la diminution du courant électrique pour déterminer la concentration de ladite séquence cible de nucléotides dans ledit échantillon.

4. Méthode d'identification selon l'une quelconque des revendications 1 à 3, **caractérisée en ce qu'**elle comprend en outre les étapes suivantes :
- on fournit de l'énergie thermique audit échantillon pour provoquer la libération dudit composé oxydo-réductible intercalé, et on applique un champ électrique audit échantillon pour enregistrer simultanément les variations de courant électrique qui traverse ledit échantillon ; et,
- on détermine la quantité d'énergie thermique Q correspondant aux variations maximales du courant électrique enregistrées pour identifier la nature de ladite séquence cible de nucléotides déterminée.

5. Méthode d'identification selon la revendication 4, **caractérisée en ce qu'**on fournit de l'énergie thermique audit échantillon de manière à provoquer une augmentation progressive de la température dudit échantillon.

6. Méthode d'identification selon la revendication 5, **caractérisée en ce qu'**on provoque l'augmentation progressive de la température entre 40°C et 98 °C.

7. Méthode d'identification selon l'une quelconques des revendications 1 à 6, **caractérisée en ce qu'**on mesure le courant électrique représentatif de l'activité électrochimique dudit composé oxydo-réductible à une température prédéterminée dudit échantillon.

8. Méthode d'identification selon les revendications 5 ou 6 et 7, **caractérisée en ce que** ladite température prédéterminée est sensiblement inférieure à la température dudit échantillon correspondant à la quantité d'énergie thermique Q déterminée.

9. Méthode d'identification selon l'une quelconque des revendications 1 à 8, **caractérisée en ce qu'**on utilise la voltampérométrie pour mesurer ledit courant électrique.

10. Méthode d'identification selon la revendication 9, **caractérisée en ce qu'**on utilise la voltampérométrie à vague carrée.

11. Méthode d'identification selon l'une quelconque des revendications 1 à 10, **caractérisée en ce que** ledit composé oxydo-réductible fourni est un composé organique intercalant de l'ADN.

12. Méthode d'identification selon l'une quelconque des revendications 1 à 10, **caractérisée en ce que** ledit composé oxydo-réductible fourni est un complexe d'un métal de transition.

13. Méthode d'identification selon la revendication 12, **caractérisée en ce que** ledit composé oxydo-réductible présente au moins un ligand intercalant de séquence d'acide nucléique.

14. Méthode d'identification selon la revendication 12 ou 13, **caractérisée en ce que** ledit composé oxydo-réductible présente le ligand dipyridophenazine.

15. Méthode d'identification selon l'une quelconque des revendications 12 à 14, **caractérisée en ce que** ledit composé oxydo-réductible présente au moins un ligand bipyridine.

16. Méthode d'identification selon l'une quelconque des revendications 1 à 15, **caractérisée en ce que** ledit composé oxydo-réductible ne vient pas oxyder les bases nucléotidiques des nucléotides libres.

17. Méthode d'identification selon l'une quelconque des revendications 1 à 16, **caractérisée en ce que** lesdits moyens d'amplification activables sont de type PCR.

18. Ensemble d'identification électrochimique de séquences cibles de nucléotides, ledit ensemble comprenant :
- des moyens (12, 14) pour recevoir un échantillon biologique susceptible de contenir une séquence cible de nucléotides déterminée ;
- des moyens d'amplification activables comportant des nucléotides libres, pour provoquer la réplication de ladite séquence cible de nucléotides déterminée et la formation de séquences cibles de nucléotides répliquées ;
- un composé oxydo-réductible apte à réagir vis-à-vis desdits nucléotides, ledit composé oxydo-réductible étant mis en contact avec ledit échantillon biologique ;
- des moyens d'activation (32, 34, 36) pour activer lesdits moyens d'amplification activables ;
- des moyens (20, 22, 24, 26, 28, 36) pour appliquer un champ électrique audit échantillon de façon à activer ledit composé oxydo-réductible et des moyens de mesure du courant électrique représentatif de l'activité électrochimique dudit composé oxydo-réductible, qui traverse ledit échantillon ; et,
- des moyens (24, 36) pour déterminer la présence de ladite séquence cible de nucléotides déterminée si le courant électrique diminue ;
**caractérisé en ce que** ledit composé oxydo-réductible est choisi parmi les composés aptes à venir s'intercaler durant la réplication entre les nucléotides formant lesdites séquences cibles répliquées, lesdites séquences cibles répliquées provoquant l'inhibition de l'activité électrochimique dudit composé oxydo-réductible intercalé, par quoi le courant électrique diminue.

19. Ensemble d'identification selon la revendication 18, **caractérisé en ce que** lesdits moyens d'activation (32, 34, 36) sont adaptés à activer lesdits moyens d'amplification activables selon des cycles d'amplification successifs pour dupliquer ladite séquence cible répliquée à chaque cycle d'amplification.

20. Ensemble d'identification selon la revendication 19, **caractérisé en ce qu'**il comprend en outre des moyens d'enregistrement (36) pour enregistrer le nombre de cycle d'amplification correspondant à la diminution du courant électrique de façon à déterminer la concentration de ladite séquence cible de nucléotides dans ledit échantillon

21. Ensemble d'identification selon l'une quelconque des revendications 18 à 20, **caractérisé en ce qu'**il comprend en outre :
- des moyens pour fournir de l'énergie thermique (32) audit échantillon de façon à provoquer la libération dudit composé oxydo-réductible intercalé, et des moyens (20, 22, 24, 26, 28, 36) pour appliquer un champ électrique audit échantillon de manière à enregistrer simultanément les variations de courant électrique qui traverse ledit échantillon ; et,
- des moyens pour déterminer la quantité d'énergie thermique correspondant aux variations maximales du courant électrique enregistrées pour identifier la nature de ladite séquence cible de nucléotides déterminée.

## Patentansprüche

1. Verfahren zur elektrochemischen Identifizierung von Zielnucleotidsequenzen, wobei das Verfahren gemäß dem nachstehenden Typ abläuft, bei dem
- man eine biologische Probe bereitstellt, in der eine bestimmte Zielnucleotidsequenz enthalten sein kann;
- man aktivierbare Amplifikationsmittel bereitstellt, die freie Nucleotide enthalten, um die Replikation der bestimmten Zielnucleotidsequenz und die Bildung von replizierten Zielnucleotidsequenzen in der Form von doppelsträngigen Nucleinsäuren hervorzurufen;
- man eine oxido-reduzierbare Verbindung bereitstellt, die mit den Nucleotiden reagieren kann, und diese oxido-reduzierbare Verbindung in Kontakt mit der biologischen Probe bringt;
- man die aktivierbaren Amplifikationsmittel aktiviert;
- man ein elektrisches Feld an die Probe anlegt, um die oxido-reduzierbare Verbindung zu aktivieren, und man den durch die Probe laufenden elektrischen Strom misst, der für die elektrochemische Aktivität dieser oxido-reduzierbaren Verbindung repräsentativ ist; und
- man die Anwesenheit der bestimmten Zielnucleotidsequenz festellt, wenn der elektrische Strom abnimmt;
**dadurch gekennzeichnet, dass** man eine oxido-reduzierbare Verbindung bereitstellt, die sich während der Replikation in die Nucleotide, die die replizierten Zielsequenzen bilden, einlagert, wobei die replizierten Zielsequenzen die Hemmung der elektrochemischen Aktivität der eingelagerten oxido-reduzierbaren Verbindung bewirken, wodurch der elektrochemische Strom abnimmt.

2. Verfahren zur Identifizierung nach Anspruch 1, **dadurch gekennzeichnet, dass** man die aktivierbaren Amplifikationsmittel gemäß aufeinanderfolgenden Amplifikationszyklen aktiviert und dass man bei jedem Amplifikationszyklus die replizierte Zielsequenz dupliziert.

3. Verfahren zur Identifizierung nach Anspruch 2, **dadurch gekennzeichnet, dass** man die Anzahl der Amplifikationszyklen entsprechend der Verringerung des elektrischen Stroms erfasst, um die Konzentration der Zielnucleotidsequenz in der Probe zu bestimmen.

4. Verfahren zur Identifizierung nach einem der Ansprüche 1 bis 3, **dadurch gekennzeichnet, dass** das Verfahren ferner die folgenden Stufen umfasst:
- man führt der Probe thermische Energie zu, um die Freisetzung der eingelagerten oxido-reduzierbaren Verbindung hervorzurufen, und man legt an die Probe ein elektrisches Feld an, um gleichzeitig die Veränderungen des durch die Probe laufenden elektrischen Stroms zu erfassen; und
- man bestimmt den Betrag der thermischen Energie Q entsprechend den maximalen erfassten Veränderungen des elektrischen Stroms, um die Art der bestimmten Zielnucleotidsequenz zu identifizieren.

5. Verfahren zur Identifizierung nach Anspruch 4, **dadurch gekennzeichnet, dass** man der Probe die thermische Energie so zuführt, dass eine zunehmende Erhöhung der Temperatur der Probe hervorgerufen wird.

6. Verfahren zur Identifizierung nach Anspruch 5, **dadurch gekennzeichnet, dass** man eine zunehmende Erhöhung der Temperatur zwischen 40 und 98 °C hervorruft.

7. Verfahren zur Identifizierung nach einem der Ansprüche 1 bis 6, **dadurch gekennzeichnet, dass** man den elektrischen Strom misst, der für die elektrochemische Aktivität der oxido-reduzierbaren Verbindung bei einer vorbestimmten Temperatur der Probe repräsentativ ist.

8. Verfahren zur Identifizierung nach den Ansprüchen 5 oder 6 und 7, **dadurch gekennzeichnet, dass** die vorbestimmte Temperatur deutlich unter der Temperatur der Probe entsprechend dem Betrag der bestimmten thermischen Energie Q liegt.

9. Verfahren zur Identifizierung nach einem der Ansprüche 1 bis 8, **dadurch gekennzeichnet, dass** man sich zur Messung des elektrischen Stroms der Voltamperometrie bedient.

10. Verfahren zur Identifizierung nach Anspruch 9, **dadurch gekennzeichnet, dass** man sich der Voltamperometrie mit quadratischer Wellenform bedient.

11. Verfahren zur Identifizierung nach einem der Ansprüche 1 bis 10, **dadurch gekennzeichnet, dass** es sich bei der bereitgestellten oxido-reduzierbaren Verbindung um eine organische Verbindung handelt, die sich in die DNA einlagert.

12. Verfahren zur Identifizierung nach einem der Ansprüche 1 bis 10, **dadurch gekennzeichnet, dass** es sich bei der oxido-reduzierbaren Verbindung um einen Übergangsmetallkomplex handelt.

13. Verfahren zur Identifizierung nach Anspruch 12, **dadurch gekennzeichnet, dass** die oxido-reduzierbare Verbindung mindestens einen Liganden aufweist, der sich in die Nucleinsäuresequenz einlagert.

14. Verfahren zur Identifizierung nach Anspruch 12 oder 13, **dadurch gekennzeichnet, dass** die oxido-reduzierbare Verbindung den Dipyridophenazin-Liganden aufweist.

15. Verfahren zur Identifizierung nach einem der Ansprüche 12 bis 14, **dadurch gekennzeichnet, dass** die oxido-reduzierbare Verbindung mindestens einen Bipyridin-Liganden aufweist.

16. Verfahren zur Identifizierung nach einem der Ansprüche 1 bis 15, **dadurch gekennzeichnet, dass** die oxido-reduzierbare Verbindung die Nucleotidbasen der freien Nucleotide nicht oxidiert.

17. Verfahren zur Identifizierung nach einem der Ansprüche 1 bis 16, **dadurch gekennzeichnet, dass** die aktivierbaren Amplifikationsmittel vom PCR-Typ sind.

18. Anordnung zur elektrochemischen Identifizierung von Zielnucleotidsequenzen, wobei die Anordnung Folgendes umfasst:
- Mittel (12, 14) zur Aufnahme einer biologischen Probe, die eine bestimmte Zielnucleotidsequenz enthalten kann;
- aktivierbare Amplifikationsmittel, die freie Nucleotide enthalten, um die Replikation der bestimmten Zielnucleotidsequenz und die Bildung von replizierten Zielnucleotidsequenzen hervorzurufen;
- eine oxido-reduzierbare Verbindung, die zur Reaktion mit den Nucleotiden befähigt ist, wobei die oxido-reduzierbare Verbindung in Kontakt mit der biologischen Probe gebracht wird;
- Aktivierungsmittel (32, 34, 36) zur Aktivierung der aktivierbaren Amplifikationsmittel;
- Mittel (20, 22, 24, 26, 28, 36) zum Anlegen eines elektrischen Felds an die Probe in der Weise, dass die oxido-reduzierbare Verbindung aktiviert wird, und Mittel zum Messen des durch die Probe laufenden elektrischen Stroms, der repräsentativ für die elektrochemische Aktivität der oxido-reduzierbaren Verbindung ist; und
- Mittel (24, 36) zur Bestimmung des Vorliegens der bestimmten Zielnucleotidsequenz, wenn der elektrische Strom abnimmt;
**dadurch gekennzeichnet, dass** die oxido-reduzierbare Verbindung unter Verbindungen ausgewählt ist, die dazu befähigt sind, sich während der Replikation in die Nucleotide, die die replizierten Zielsequenzen bilden, einzulagern, wobei die replizierten Zielsequenzen eine Hemmung der elektrochemischen Aktivität der eingelagerten oxido-reduzierbaren Verbindung hervorrufen, wodurch der elektrische Strom abnimmt.

19. Anordnung zur Identifizierung nach Anspruch 18, **dadurch gekennzeichnet, dass** die Aktivierungsmittel (32, 34, 36) zur Aktivierung der aktivierbaren Amplifikationsmittel gemäß den aufeinanderfolgenden Amplifikationszyklen zur Duplizierung der replizierten Zielsequenz bei jedem Amplifikationszyklus in der Lage sind.

20. Aordnung zur Identifizierung nach Anspruch 19, **dadurch gekennzeichnet, dass** sie ferner Aufzeichnungsmittel (36) umfasst, um die Anzahl der Amplifikationszyklen entsprechend der Abnahme des elektrischen Stroms zu bestimmen, um die Konzentration der Zielnucleotidsequenz in der Probe zu erfassen.

21. Anordnung zur Identifizierung nach einem der Ansprüche 18 bis 20, **dadurch gekennzeichnet, dass** sie ferner Folgendes umfasst:
- Mittel zur Versorgung der Probe mit thermischer Energie (32), um eine Freisetzung der eingelagerten oxido-reduzierbaren Verbindung hervorzurufen, und Mittel (20, 22, 24, 26, 28, 36), um ein elektrisches Feld an die Probe anzulegen, um gleichzeitig die Veränderungen des durch die Probe laufenden elektrischen Stroms zu erfassen; und
- Mittel zur Bestimmung des Betrags der thermischen Energie, entsprechend den erfassten maximalen Veränderungen des elektrischen Stroms, um die Art der bestimmten Zielnucleotidsequenz zu identifizieren.

## Claims

1. A method for electrochemically identifying target nucleotide sequences, said method being of the type according to which:
- a biological sample that may contain a predetermined target nucleotide sequence is provided;
- activatable amplification means comprising free nucleotides in order to cause the replication of said predetermined target nucleotide sequence and the formation of replicated target nucleotide sequences are provided in the form of nucleic acid double strands;
- an oxido-reducible compound capable of reacting with respect to said nucleotides is provided and said oxido-reducible compound is brought into contact with said biological sample;
- said activatable amplification means are activated;
- an electric field is applied to said sample in order to activate said oxido-reducible compound and the electric current representative of the electrochemical activity of said oxido-reducible compound, which passes through said sample, is measured; and
- the presence of said predetermined target nucleotide sequence is determined if the electric current decreases;
**characterized in that** an oxido-reducible compound capable of intercalating, during the replication, between the nucleotides forming said replicated target sequences is provided, said replicated target sequences causing the inhibition of the electrochemical activity of said intercalated oxido-reducible compound, by virtue of which the electric current decreases.

2. The identification method as claimed in claim 1, **characterized in that** said activatable amplification means are activated according to successive amplification cycles and **in that**, at each amplification cycle, said replicated target sequence is duplicated.

3. The identification method as claimed in claim 2, **characterized in that** the number of amplification cycles corresponding to the decrease in the electric current is recorded in order to determine the concentration of said target nucleotide sequence in said sample.

4. The identification method as claimed in any one of claims 1 to 3, **characterized in that** it also comprises the following steps:
- said sample is supplied with thermal energy in order to cause the release of said intercalated oxido-reducible compound, and an electric field is applied to said sample in order to simultaneously record the variations in electric current which passes through said sample; and
- the amount of thermal energy Q corresponding to the maximum variations in the electric current which are recorded is determined in order to identify the nature of said predetermined target nucleotide sequence.

5. The identification method as claimed in claim 4, **characterized in that** said sample is supplied with thermal energy so as to cause a gradual increase in the temperature of said sample.

6. The identification method as claimed in claim 5, **characterized in that** the gradual increase in the temperature is caused between 40°C and 98°C.

7. The identification method as claimed in any one of claims 1 to 6, **characterized in that** the electric current representative of the electrochemical activity of said oxido-reducible compound is measured at a predetermined temperature of said sample.

8. The identification method as claimed in claims 5 or 6 and 7, **characterized in that** said predetermined temperature is noticeably lower than the temperature of said sample corresponding to the predetermined amount of thermal energy Q.

9. The identification method as claimed in any one of claims 1 to 8, **characterized in that** voltammetry is used for measuring said electric current.

10. The identification method as claimed in claim 9, **characterized in that** square wave voltammetry is used.

11. The identification method as claimed in any one of claims 1 to 10, **characterized in that** said oxido-reducible compound provided is a DNA-intercalating organic compound.

12. The identification method as claimed in any one of claims 1 to 10, **characterized in that** said oxido-reducible compound is a complex of a transition metal.

13. The identification method as claimed in claim 12, **characterized in that** said oxido-reducible compound has at least one nucleic-acid-sequence-intercalating ligand.

14. The identification method as claimed in claims 12 or 13, **characterized in that** said oxido-reducible compound has the dipyridophenazine ligand.

15. The identification method as claimed in any one of claims 12 to 14, **characterized in that** said oxido-reducible compound has at least one bipyridine ligand.

16. The identification method as claimed in any one of claims 1 to 15, **characterized in that** said oxido-reducible compound does not oxidize the nucleotide bases of the free nucleotides.

17. The identification method as claimed in any one of claims 1 to 16, **characterized in that** said activatable amplification means are of PCR type.

18. An assembly for electrochemically identifying target nucleotide sequences, said assembly comprising:
- means (12, 14) for receiving a biological sample that may contain a predetermined target nucleotide sequence;
- activatable amplification means comprising free nucleotides in order to cause the replication of said predetermined target nucleotide sequence and the formation of replicated target nucleotide sequences;
- an oxido-reducible compound capable of reacting with respect to said nucleotides, said oxido-reducible compound being brought into contact with said biological sample;
- activating means (32, 34, 36) for activating said activatable amplification means;
- means (20, 22, 24, 26, 28, 36) for applying an electric field to said sample so as to activate said oxido-reducible compound, and means for measuring the electric current representative of the electrochemical activity of said oxido-reducible compound, which passes through said sample; and
- means (24, 36) for determining the presence of said predetermined target nucleotide sequence if the electric current decreases;
**characterized in that** said oxido-reducible compound is chosen from compounds capable of intercalating, during the replication, between the nucleotides forming said replicated target sequences, said replicated target sequences causing the inhibition of the electrochemical activity of said intercalated oxido-reducible compound, by virtue of which the electric current decreases.

19. The identification assembly as claimed in claim 18, **characterized in that** said activating means (32, 34, 36) are suitable for activating said activatable amplification means according to successive amplification cycles in order to duplicate said replicated target sequence at each amplification cycle.

20. The identification assembly as claimed in claim 19, **characterized in that** it also comprises recording means (36) for recording the number of amplification cycles corresponding to the decrease in the electric current so as to determine the concentration of said target nucleotide sequence in said sample.

21. The identification assembly as claimed in any one of claims 17 to 20, **characterized in that** it also comprises:
- means (32) for supplying said sample with thermal energy so as to cause the release of said intercalated oxido-reducible compound, and means (20, 22, 24, 26, 28, 36) for applying an electric field to said sample so as to simultaneously record the variations in electric current which passes through said sample; and
- means for determining the amount of thermal energy corresponding to the maximum variations in the electric current which are recorded in order to identify the nature of said predetermined target nucleotide sequence.
